# EUROPEAN PATENT SPECIFICATION

(11) **EP 3 003 182 B1**
(45) Date of publication and mention of the grant of the patent: **26.10.2016**
(21) Application number: 14726129.1
(22) Date of filing: 21.05.2014
(51) Int. Cl.: A61B 17/54, A61H 9/00, A61B 17/00, A61B 17/30, A61B 18/00

(54) **NON-INVASIVE DEVICE FOR REJUVENATION OF SKIN TISSUE USING TREATMENT PRESSURE BELOW AMBIENT PRESSURE**
NICHTINVASIVE VORRICHTUNG ZUR VERJÜNGUNG DES HAUTGEWEBES MIT BEHANDLUNGSDRUCK UNTER UMGEBUNGSDRUCK
DISPOSITIF NON INVASIF POUR LE RAJEUNISSEMENT DE LA PEAU DES TISSUS À L'AIDE DE LA PRESSION DE TRAITEMENT SOUS PRESSION AMBIANTE

(30) Priority: 30.05.2013 EP 13169832
(43) Date of publication of application: 13.04.2016
(73) Proprietor: Koninklijke Philips N.V., 5656 AE Eindhoven (NL)
(72) Inventor: HORTON, Margaret Ruth, NL-5656 AE Eindhoven (NL); JURNA, Martin, NL-5656 AE Eindhoven (NL); PALERO, Jonathan Alambra, NL-5656 AE Eindhoven (NL); VARGHESE, Babu, NL-5656 AE Eindhoven (NL)
(74) Representative: Freeke, Arnold
(86) International application number: PCT/EP2014/060379
(87) International publication number: WO 2014/191263

(56) References cited:
- WO-A1-2011/021184
- WO-A2-2007/117580
- DE-A1-102011 102 387

## Description

### FIELD OF THE INVENTION

This invention relates to a non-invasive device for rejuvenation of the skin tissue using a treatment pressure below ambient pressure.

This invention further relates to a method for rejuvenation of skin tissue.

### BACKGROUND OF THE INVENTION

Skin rejuvenation is an intervention that generates new skin tissue to replace aged or damaged skin tissue. Typically, the epidermis layer of the skin tissue is a target of skin rejuvenation to specifically improve the skin tone and clarity. However, also the dermis layer of the skin tissue may be the target. Rejuvenation of the epidermis layer most commonly involves removing or damaging part of the epidermis layer to stimulate the skin's generation of new epidermis tissue. This is particularly effective for achieving, for example, a more even skin tone because melanin in the epidermis layer may cause a mottled or uneven skin tone.

Damaging the epidermis layer may be achieved, for example, mechanically. with microdermabrasion, chemically using a chemical peel, or optically using a light or laser source that is preferentially superficially absorbed, for example, by the water located in the epidermis (such as with a 1927-nm thulium laser). Also damage to the dermis layer may be used for skin rejuvenation which may, for example, be achieved via thermal damages to the dermis layer or, for example, using focused laser light to create laser induced optical breakdown (LIOB) inside the dermis layer, e.g., known from the published international patent application WO 2008/001284 A2.

As to the different methods of damaging the epidermis layer or the dermis layer in the skin tissue to initiate skin rejuvenation, the non-invasive methods are preferred as they significantly reduce any risk of infection and complications. Still, some of the known non-invasive methods may be relatively painful.

WO 2011/021184 A1 discloses a method and an apparatus for effecting vacuum and massage treatment to human skin tissue for the reduction of cellulite. The method and apparatus are based on coupling an applicator accommodating at least two vacuum chambers, sharing one or more common walls there between, to the surface of the skin and alternately reducing the air pressure in the vacuum chambers to affect vacuum suction to the skin, thereby alternately drawing adjacent segments of skin into the vacuum chambers. This alternating suction effect generates enhanced massaging and forth movement of the skin tissue against the common wall between adjacent vacuum chambers, parallel to the skin surface and perpendicular to the collagen fibrous septae orientation in the sub-dermis skin tissue. The preamble of independent claims 1 and 15 is based on WO 2011/021184 A1.

WO 2007/117580 A2 discloses a method for treating a volume of skin tissue, wherein a negative pressure is applied to at least a portion of the volume of skin tissue, wherein a second portion of the volume of skin tissue is mechanically restrained, and wherein the second portion of the volume of skin tissue is irradiated with light. As a result of the application of the negative pressure the skin target is maintained under tension so as to redistribute the blood volume between the skin target and other skin segments. Such tension causes a depletion of the volumetric blood content in the skin target, which facilitates the delivery of the treatment light to the skin target.

DE 10 2011 102 387 A1 discloses a simple and improved cupping applicator for connection to a vacuum source, having a treatment opening that is partially covered by a flat template with a contoured break-through such that a skin is sucked during the cupping process. The applicator has a treatment opening with a diameter of 25 millimeter. The break-through is provided with a clover sheet.

### OBJECT OF THE INVENTION

It is an object of the invention to provide a non-invasive device for skin rejuvenation enabling the skin rejuvenation treatment to be less painful.

### SUMMARY OF THE INVENTION

A first aspect of the invention provides a non-invasive device for rejuvenation of skin tissue comprising a suction chamber. A second aspect of the invention provides a skin treatment method.

The non-invasive device for rejuvenation of skin tissue according to the first aspect of the invention comprises a suction chamber having a skin contact surface which comprises an opening for exposing the skin tissue to a treatment pressure present in the suction chamber for applying a suction force to a portion of an outer surface of the skin tissue corresponding to an area of the opening. The treatment pressure is lower than an ambient pressure outside the suction chamber, and the suction chamber is dimensioned so as to allow it to be manually applied to the outer surface of the skin tissue. The area of the opening in the suction chamber is less than 10 square millimeters. The non-invasive device further comprises a controller for controlling a level of the treatment pressure inside the suction chamber and for controlling an application time interval of the treatment pressure. This combination of the treatment pressure inside the suction chamber and the application time interval damages an interface between an epidermis layer and a dermis layer of the skin tissue to initiate rejuvenation of the skin tissue. Damaging the interface between the epidermis layer and the dermis layer includes causing small, even microscopic, tears and ruptures which may generate small vacuoles that are or become filled with moisture. Damaging the interface between the epidermis layer and the dermis layer also includes separating the epidermis layer and the dermis layer, which typically results in a blister formed at the separated location.

Due to the use of the relatively small opening through which the suction force is applied to the outer surface of the skin tissue, the use of the non-invasive device on a patient has been proven to be substantially painless, involving a very low discomfort level while the damaging of the interface between the epidermis layer and the dermis layer initiates the generation of new epidermal tissue, which results in skin rejuvenation. A further effect of the non-invasive device is that the damaging or separation of the interface between the epidermis layer and the dermis layer over a relatively small area, such as the relatively small opening in the skin contact surface, ensures that there is enough healthy and undamaged or unseparated skin tissue surrounding the damaged part of the skin tissue. This relatively small damaged interface in the skin tissue is sufficient to initiate the skin rejuvenation while the presence of the surrounding healthy skin tissue further stimulates and speeds up the process of generating new epidermal tissue and therefore speeds up the rejuvenation process.

As mentioned before, the use of a non-invasive device for rejuvenation treatment has the benefit that any risk of infections and complications is reduced significantly. In addition, the non-invasive device according to the invention further uses a relatively small opening area for applying the treatment pressure to the outer skin, such that any surface effects such as discoloration or blister formation will occur only at the location of that relatively small opening in the skin contact surface. This clearly limits any cosmetic and visual effects of the treatment when using the non-invasive device according to the invention. Finally, due to the use of the relatively small opening area through which the suction force is applied to the outer surface of the skin, the treatment time required to generate the necessary damage at the interface between the epidermis layer and the dermis layer is significantly reduced. The inventors have found that the treatment time to induce the necessary damage at the interface between the epidermis layer and the dermis layer is substantially linear with a width of the opening in the skin contact surface of the suction chamber. Therefore, using the relatively small opening as defined in the current non-invasive device enables such pressure treatment processes to be applied within acceptable time limits for such a rejuvenation treatment. Further reduction of the opening would further reduce the treatment time.

The opening may, for example, be a symmetrical opening such as a substantially round opening having a diameter of less than approximately 3.5 millimeters, or, for example, a substantially square opening having approximate dimensions of less than 3 millimeters by 3 millimeters. However, the opening may have any shape such as rectangular or elongated. Around the opening, the skin contact surface should have an area which is free from any further openings to ensure that sufficient healthy tissue surrounds the treated part of the skin tissue. The skin contact surface may be curved or may have a shape which, for example, mimics contours of the body on which the non-invasive device may be applied for rejuvenating skin tissue. For example, the skin contact surface may be a kind of mask or partial mask that fits on a face and which has one or a plurality of openings at locations where the skin tissue rejuvenation process may be applied. Alternatively, the skin contact surface may, for example, have a shape like a kind of glove to carry out the rejuvenation process on parts of a hand. The controller may be any controller capable of controlling the level of the treatment pressure and the application time interval. The application time interval may be controlled simply by providing an alarm after the application time interval has lapsed, so that the user is alarmed to stop the application of the treatment pressure applied by the non-invasive device according to the invention. Alternatively, the controller may stop the rejuvenation treatment automatically after lapse of the application time interval. It should be noted that the damaging of the interface between the epidermis layer and the dermis layer may lead to blisters, and that the generation of a blister at the location of the opening initiates the skin rejuvenation, but the generation of a blister or a reference blister is not essential to initiate the skin rejuvenation process. Creating some damage or a degree of separation at the interface between the epidermis layer and the dermis layer may contain microscopic tears and ruptures which generate small vacuoles, for example, having a diameter of approximately 20 microns, which are filled with moisture. These small vacuoles are proof of damage or separation created at the interface between the epidermis layer and the dermis layer and they occur before a blister or reference blister is formed at the treatment location. Still, inducing such damage at the treatment location by the non-invasive device according to the invention would be sufficient to initiate the rejuvenation process inside the skin tissue while hardly any visual effects of the treatment are present at the surface of the skin. The reference blister or reference blister formation corresponds to a blister formation comprising one or more blister cavities at the interface between the epidermis layer and the dermis layer covering 80% of the area of the opening (120) in the suction chamber. Typically, such a cavity is also filled with fluid. The presence of such microscopic damages or separation at the interface between the epidermis layer and the dermis layer may be identified via visual inspection of a pigmented area of the skin tissue which is, for example, partly being treated using the non-invasive device according to the invention. The coloring in such a pigmented area may change (decrease or become more homogeneous) due to the treatment using the non-invasive device- such a coloring change may occur after 14 to 28 days. An alternative way to identify the presence of such microscopic damages or separation may be by using confocal microscopy in which the interface between the epidermis layer and dermis layer is investigated. Before the treatment, the interface between the epidermis layer and the dermis layer may be relatively even and flat, while after the treatment using the non-invasive device according to the invention, the interface may become more undulated due to the creation of new epidermis tissue at the interface - which may occur after 30 to 90 days. A third method of identifying the presence of the microscopic damage would be to use optical coherence tomography in which again the difference between the initial flat interface between the epidermis layer and the dermis layer before treatment may be compared with the undulated characteristic of the interface after the treatment using the non-invasive device according to the invention. Using optical coherence tomography, the separation and/or vacuole formation may be visible almost immediately after treatment.

To ensure that the treatment pressure can be used to apply the suction force to the outer surface of the skin tissue, the skin contact surface may be made of a material capable of forming a vacuum seal on the surface of the skin. Such a material may, for example, comprise aluminum metal or elastomeric polymer material. The suction chamber has a dimension that allows the suction chamber to be manually applied to the outer surface of the skin tissue. The suction chamber may be connected to a vacuum pump, for example, via a tube to limit the overall weight of the non-invasive rejuvenation device such that it may easily be handled and manually applied to the outer surface of the skin tissue. Of course, alternatively, if the vacuum pump is relatively small and lightweight, the vacuum pump may be integrated in an overall non-invasive rejuvenation device according to the invention while the device can still be manually applied to the outer surface of the skin tissue. An overall weight above 20 kilograms may be too heavy for manually applying such a device. The outer dimensions of the suction chamber may resemble, for example, a mask to be applied to a face of a person undergoing the treatment, or, alternatively, the outer dimensions of the suction chamber may resemble part of a hand, arm, leg or other part of the human body to which the rejuvenation treatment using the non-invasive device according to the invention may be applied. In addition, the use of lightweight materials to produce the suction chamber may further contribute to the ability to manually apply the suction chamber to the outer surface of the skin tissue.

In an embodiment of the non-invasive device according to the invention, the opening in the skin contact surface of the suction chamber is in a range between 4 square millimeters and 0.75 square millimeter. As mentioned before, there is an almost linear relationship between the width of the opening and the time required to generate damage or even to generate a blister between the epidermis layer and the dermis layer. Further reducing the dimensions of the opening may further reduce the required treatment time. Of course, when reducing the dimensions of the opening too much, the treatment pressure may not really damage or separate the interface between the epidermis layer and the dermis layer, as the mechanical deformation due to the treatment pressure may not be sufficient. As no damage or separation occurs, the rejuvenation process is not initiated when the openings used are too small.

In an embodiment of the non-invasive device according to the invention, the level of the treatment pressure in the suction chamber is in a range between 0.1333x10⁵ and 1.2x10⁵ N/m² (between 10⁵ and 9x10⁵ milliTor) below ambient pressure. 10⁵ milliTor is substantially equal to 100 millimeter Mercury (at 0° Celsius) which is substantially equal to 13.33 kiloPascal. 9x10⁵ milliTor is substantially equal to 900 millimeter Mercury (at 0° Celsius) which is substantially equal to 120 kiloPascal. Further reducing the pressure (or reducing the treatment pressure) below ambient pressure will reduce the time required to damage the interface between the epidermis layer and the dermis layer.

In an embodiment of the non-invasive device according to the invention, the application time interval is in a range between 1 second and 10 minutes. As already has been made clear hereinabove, the exact application time interval used for the treatment by the non-invasive device according to the invention may strongly depend on the dimension of the opening in the skin contact surface together with the level of treatment pressure applied in the suction chamber. Another element which determines the preferred application time interval for the treatment is whether a blister or reference blister formation is to be generated or that blister forming should be avoided and that only microscopic damage is to be generated. Finally, skin type, age and location on, for example, a human body determine the required combination of application time interval, treatment pressure and the area of the opening in the skin contact surface to achieve the necessary level of damage at the interface of the epidermis layer and the dermis layer to efficiently initiate skin rejuvenation. For example, a reference blister-time is a time interval during which, using a predetermined area of the opening and using a predefined level of treatment pressure in the suction chamber, the treatment pressure should be applied for creating the reference blisterformation. This reference blister-time may be experimentally determined at one location on the human body when using a specific level of treatment pressure and a specific area of the opening in the skin contact surface. This reference blister-time may subsequently be used to determine the application time interval set at the controller for maintaining the level of treatment pressure during the treatment using the non-invasive device according to the invention.

In an embodiment of the non-invasive device, the skin contact surface comprises an array of openings, a distance between two adjacent openings in the array of openings being larger than a width of the opening. The distance between two adjacent openings and the width of the opening should be measured substantially along the same imaginary line drawn between two adjacent openings. A benefit of this embodiment is that the array of openings ensures that a single treatment step using the non-invasive device according to the invention induces damage at the interface between the epidermis layer and the dermis layer at multiple locations on the skin surface, and the distance requirement between two adjacent openings ensures that sufficient healthy tissue surrounds any damaged tissue to initiate an efficient rejuvenation process. Such fractional treatment has been proven to be very efficient both by speeding up the overall rejuvenation process because of the presence of the healthy tissue and by increasing the overall treatment area due to the array of openings.

In an embodiment of the non-invasive device, the controller comprises a look-up-table for determining the level of the treatment pressure and the application time interval for a specific skin type. Such a look-up-table may, for example, indicate for different skin types or for different locations on, for example, the human body which combination of the application time interval, level of treatment pressure and dimensions of the opening in the skin contact surface may be chosen to achieve a required level of damage at the interface between the epidermis layer and the dermis layer. The look-up-table may be generated experimentally. The user may, for example, be asked to input information about his skin type, such that the required level of treatment pressure and application time interval may be determined. Alternatively, specific characteristics of the skin may be measured - for example by the non-invasive device according to the invention or by another measurement device. Such characteristics of the skin may be epidermal thickness, elasticity, which is used to empirically determine the level of treatment pressure and the application time interval. Even further alternatively, a user may use the look-up-table provided with the non-invasive device for determining the application time interval and the level of treatment pressure.

In an embodiment of the non-invasive device, the non-invasive device comprises a sensor connected to the controller, the sensor being configured for sensing a parameter associated with the damaging of the interface between an epidermis layer and a dermis layer of the skin tissue. The controller may use the information provided by the sensor to determine the application time interval during which the treatment pressure is to be applied to the surface of the skin. As such, the sensor may simply be used to indicate to the controller when to stop the treatment. One embodiment of such a sensor may, for example, be a pressure sensor. Such a pressure sensor may, for example, provide a signal to the controller representing the pressure inside the suction chamber. The controller may, for example, analyze the variation of the treatment pressure inside the suction chamber and when the level of treatment pressure abruptly reduces (i.e. gets closer to the ambient pressure level), and this may be an indicator that the interface between the epidermis layer and the dermis layer has been separated or damaged and that treatment may be stopped at that particular location on the skin. Alternatively, the pressure sensor may analyze the variation of the treatment pressure in the suction chamber and, when this variation occurs, provide a signal to the controller to stop the treatment. Alternatively, the sensor may, for example, be an optical sensor. Such an optical sensor may, for example, sense a change of the outer surface of the skin tissue such as the height of a skin dome which may be an indicator that damage or separation has occurred at the interface between the epidermis layer and the dermis layer. Alternatively, the optical sensor may also focus inside the skin and may sense a change within the skin at the interface between the epidermis layer and the dermis layer. When such a change occurs, the optical sensor may provide a signal to the controller to stop the treatment. Another alternative sensor may be an acoustic sensor. If damage occurs in the interface between the epidermis layer and the dermis layer, the microscopic rupturing and tearing at the interface will generate acoustic waves progressing through the skin tissue which may, for example, be captured using piezo-electric or other acoustic sensors, for example, touching the skin next to the opening in the skin contact layer. Using such sensors may ensure that full blisters are avoided during the treatment using the non-invasive device according to the invention.

In an embodiment of the non-invasive device, the controller is configured to stop the treatment pressure being applied to the skin tissue before a reference blister formationis formed at the interface between the epidermis layer and the dermis layer in the skin tissue. The reference blister formation corresponds to a blister formation comprising one or more blister cavities at the interface between the epidermis layer and the dermis layer covering 80% of the area of the opening in the suction chamber. Avoiding the occurrence of blisters is preferred from a cosmetic point of view and is not essential for the initiation of the rejuvenation process. A reference blister-time, for example, is a time interval during which, using a predetermined area of the opening and using a predefined level of treatment pressure in the suction chamber, the treatment pressure should be applied for creatingthe reference blister formation. The controller may be configured to stop the application of the treatment pressure after an application time interval which is within a range between 30% and 70% of said reference blister-time.

The non-invasive device according to the invention may thus be able to initiate the rejuvenation process while preventing the occurrence of blisters by using a signal from a sensor included in the non-invasive device which limits and/or determines the application time interval, or by using a portion of the reference blister-time which may, for example, be experimentally determined, or by using a look-up-table in which preferred combinations of the level of the treatment pressure, application time interval and dimensions of the opening in the skin contact surface are listed. Again, as indicated several times hereinbefore, the occurrence of blisters would not harm the rejuvenation process started by the damaging of the interface between the epidermis layer and the dermis layer, but neither is it essential for the rejuvenation process to be initiated. And from a cosmetic point of view, the prevention of full blisters during the treatment using the non-invasive device according to the invention would be preferred.

In an embodiment of the non-invasive device according to the invention, the non-invasive device comprises a heating element being configured for increasing a temperature of the skin tissue at or near the interface between the epidermis layer and the dermis layer. The increase of the temperature at or near the interface also reduces the overall treatment time required to induce damage at the interface to initiate the rejuvenation process. An increase of the temperature to 40 or 42 degrees Celsius would significantly reduce the treatment time. This reduction of time due to the increased temperature may, for example, be experimentally determined and/or may be included in a look-up-table. A non-exhaustive listing of examples of different heating elements that may be used are a Radio-Frequency (RF) heating element, a laser heating element, a Light Emitting Diode heating element, a contact heating element, a Peltier heating element and a broadband light source such as intense pulsed light from a flash lamp. Of course also other heating elements may be used.

In an embodiment of the non-invasive device, the heating element is configured for increasing the temperature of the skin tissue to a level necessary for thermally damaging the epidermis layer, dermis layer, or the interface between the epidermis layer and the dermis layer. In a previous embodiment of the non-invasive device, the temperature at the interface was increased to reduce the time required to induce the damage or separation at the interface caused by the treatment pressure applied, so that the overall treatment time was significantly reduced. However, further increasing the temperature to at least 55°C, and up to 80°C, may directly cause thermal damage in the epidermis layer, dermis layer, or at the interface. In such an embodiment, the rejuvenation is initiated using the suction force applied from the suction chamber, but in addition, the heating element may further increase the temperature to induce further damage in the skin tissue to initiate rejuvenation also via thermal injury or denaturation of proteins. Such thermal damage may be applied deeper into the skin tissue and may complement the suction treatment.

In an embodiment of the non-invasive device, the non-invasive device comprises a cooling element being configured for reducing a temperature of the skin tissue at or near the interface between the epidermis layer and the dermis layer. Such a cooling element may, for example, be a Peltier cooling element for cooling the interface to a range between -20 and -30 degrees Celsius. The inventors have found that also this reduction of the temperature at or near the interface would reduce the time required to induce sufficient damage at the interface to initiate skin rejuvenation. Also this time reduction may be experimentally determined and/or may be included in a look-up-table.

The method of rejuvenating skin tissue according to the second aspect of the invention comprises the steps of:
bringing a suction chamber into contact with an outer surface of skin tissue, the suction chamber having a skin contact surface comprising an opening for exposing the skin tissue to a treatment pressure present in the suction chamber for applying a suction force to a portion of the outer surface of the skin tissue corresponding to an area of the opening, the treatment pressure being lower than an ambient pressure outside the suction chamber, and the suction chamber being dimensioned so as to allow the suction chamber to be manually applied to the outer surface of the skin tissue, the area of the opening in the suction chamber being less than 10 square millimeters, and
applying a level of treatment pressure during an application time interval to the outer surface of the skin tissue using the suction chamber for damaging an interface between an epidermis layer and a dermis layer of the skin tissue to initiate rejuvenation of the skin tissue.

These and other aspects of the invention are apparent from and will be elucidated with reference to the embodiments described hereinafter.

### BRIEF DESCRIPTION OF THE DRAWINGS

In the drawings:
Figures 1A and 1B schematically show a non-invasive device according to the invention,
Figures 2A, 2B and 2C show different stages in the process for initiation of skin rejuvenation,
Figure 3A shows a detail of the non-invasive device according to the invention in which the non-invasive device comprises a sensor for sensing a parameter associated with the damaging of the interface between the epidermis layer and the dermis layer, and Figure 3B shows a variation of the pressure inside the suction chamber during treatment using the non-invasive device 100 according to the invention,
Figure 4 shows a detail of the non-invasive device according to the invention, in which the non-invasive device comprises a heating element or cooling element,
Figures 5A and 5B show graphs indicating the blister time Tb required for different opening widths and treatment pressure levels Pt and for different temperatures, and
Figure 6 shows a flow diagram indicating the method according to the invention and a computer program product to perform the skin treatment method according to the invention.

### DETAILED DESCRIPTION OF THE INVENTION

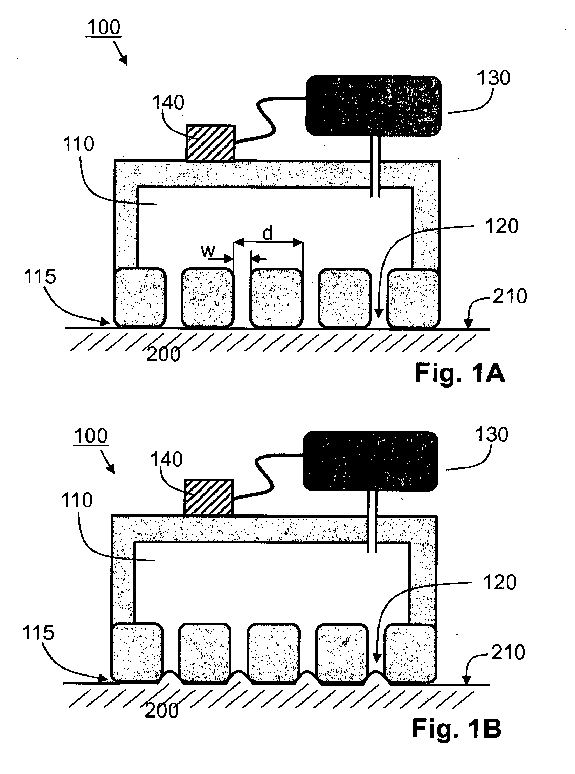

Figures 1A and 1B schematically show a non-invasive device 100 according to the invention. The non-invasive device 100 comprises a suction chamber 110 which comprises a skin contact surface 115 having at least one opening 120 for exposing skin tissue 200 to the suction chamber 110. The suction chamber 110 comprises, for example, a vacuum pump 130 or any other element 130 connected to the suction chamber 110 for reducing a pressure inside the suction chamber 110 compared to ambient pressure and hence to apply a suction force to an outer surface 210 of the skin tissue 200. The non-invasive device 100 according to the invention further comprises a controller 140 which is configured for controlling a level of the treatment pressure Pt inside the suction chamber 110 and for controlling an application time interval Δt during which the treatment pressure is to be applied to a portion of the outer surface 210 of the skin tissue 200 corresponding to an area of said opening 120. The controller 140 may, for example, be connected to the vacuum pump 130 to control the level of the treatment pressure Pt and, for example, to switch off the vacuum pump 130 after the application time interval Δt has lapsed. Alternatively, the controller 140 may provide an indicator (not shown) such as a visual or audible indicator for indicating whether the application time interval Δt has lapsed, so that a user or operator of the non-invasive device 100 according to the invention may stop the treatment at that location of the skin tissue 200.

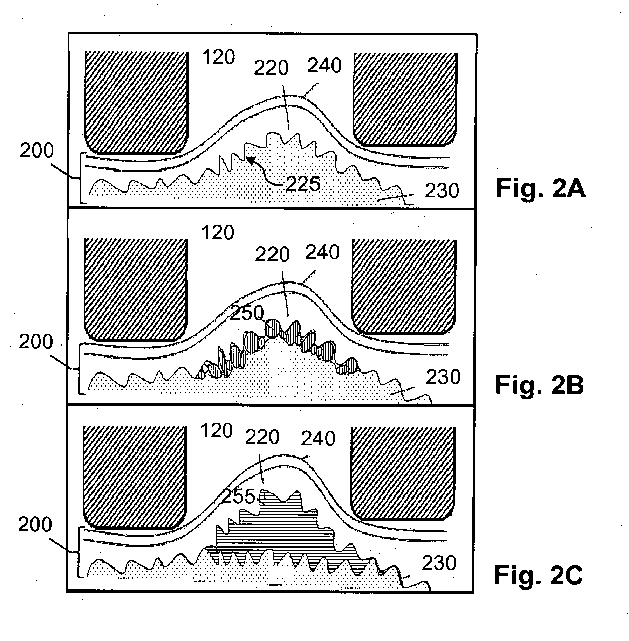

Due to the application of the suction force by the suction chamber 110 via the opening 120, an interface 225 (see Fig. 2A) between an epidermis layer 220 (see Fig. 2A) and a dermis layer 230 (see Fig. 2A) of the skin tissue 200 is damaged, which initiates a rejuvenation process of the skin tissue 200. The inventors have found that due to the use of the relatively small opening 120 through which the suction force is applied to the outer surface 210 of the skin tissue 200, the use of the non-invasive device 100 on a patient has been proven to be substantially painless, involving a very low discomfort level while damaging the interface 225 between the epidermis layer 220 and the dermis layer 230. A further effect of the non-invasive device 100 is that the damaging of the interface 225 occurs over a relatively small area (the relatively small opening 120) such that there is enough healthy and undamaged skin tissue 200 surrounding the damaged part of the skin tissue 200. Said presence of surrounding healthy skin tissue 200 further stimulates and speeds up the process of generating new epidermal tissue and hence speeds up the rejuvenation process. An even further benefit of the non-invasive device 100 according to the invention is that the outer surface 210 of the skin tissue 200 remains intact during the treatment, which reduces the risk of infections and complications. And finally, the use of the relatively small opening 120 for applying the level of treatment pressure Pt reduces any undesired cosmetic surface effects such as coloring or blister formation.

The non-invasive device according to the invention comprises an opening via which the outer surface 210 of the skin tissue 200 is exposed to the suction chamber 110. The dimensions of this opening are smaller than 10 square millimeters, and may, for example, be in a range between 4 square millimeters and 0.75 square millimeter. The opening 120 may, for example, be a symmetrical opening 120 such as a substantially round opening 120 having a diameter (or width w) of less than approximately 3.5 millimeters, or, for example, a substantially square opening 120 having approximate dimensions of less than 3 millimeters by 3 millimeters. However, the opening 120 may have any shape such as rectangular or elongated. The skin contact surface 115 may have a single opening 120, but alternatively, the skin contact surface 115 may have an array of openings 120 for applying the skin rejuvenation treatment at multiple locations on the outer surface 210 of the skin tissue 200 simultaneously. In such an array of openings 120, a distance d between two adjacent openings should be larger than a width w of the individual opening 120. A benefit of such an arrangement is that there is a relatively large area of skin tissue 200 around the opening 120 where no damaging of the interface 225 (see Fig. 2A) between the epidermis layer 220 (see Fig. 2A) and the dermis layer 230 (see Fig. 2A) occurs. As mentioned before, the presence of healthy (non-damaged) tissue around the treated area speeds up the rejuvenation process. In the embodiment shown in Figures 1A and 1B, the skin contact surface 115 is a substantially flat surface. However, the skin contact surface 115 may have any shape, such as curved, or may even have a shape which, for example, mimics contours of the body on which the non-invasive device 100 may be applied for rejuvenating skin tissue 200. For example, the skin contact surface 115 may be a kind of mask (not shown) or partial mask (not shown) which fits on the face of a person who is going to have the skin rejuvenation treatment and which has one or a plurality of openings at locations where the skin tissue 200 rejuvenation process may be applied. Alternatively, the skin contact surface 115 may, for example, be shaped like a kind of glove (not shown) to apply the rejuvenation process on parts of a hand. Alternatively, the skin contact surface 115 may be a relatively flat, relatively small surface which may, for example, be repositioned in an area to be treated after each application time interval - similar to a stamp being repositioned to cover a specific area.

Figure 1A shows the non-invasive device 100 applied to the outer surface 210 of the skin tissue 200 while no treatment pressure is applied in the suction chamber 110, and Figure 1A shows the non-invasive device 100 in which the treatment pressure Pt is applied to the exposed parts of the outer surface 210 of the skin tissue 200 via the openings 120. As is schematically shown in Figure 1B, the outer surface 210 of the skin tissue 200 is pulled into the suction chamber 110 through the opening 120, causing the damaging at the interface 225 between the epidermis layer 220 and the dermis layer 230.

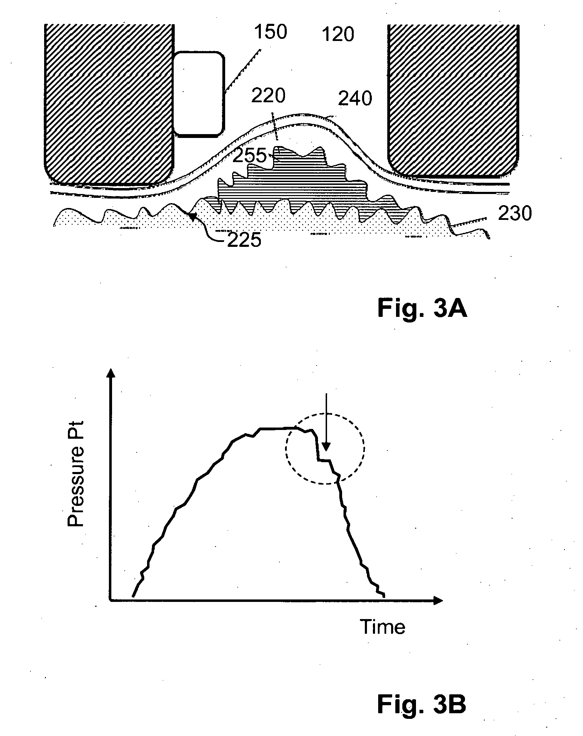

Figures 2A, 2B and 2C show different stages during the process for initiation of skin rejuvenation. Figures 2A, 2B and 2C only show part of the non-invasive device 100 according to the invention (only show a single opening 120) and show a cross-sectional detail of the damaging process inside the skin tissue 200. In the cross-sectional detail of the skin tissue 200, the stratum corneum 240 is shown, together with the epidermis layer 220 and the dermis layer 230. The damage due to applying the suction force during the application time interval Δt occurs at the interface 225 between the epidermis layer 220 and the dermis layer 230. Initially - as shown in Figure 2A - the application of the suction force to the outer surface 210 of the skin tissue 200 deforms the skin tissue 200. After some time - as shown in Figure 2B - initial separation of the interface 225 between the epidermis layer 220 and the dermis layer 230 starts to take place, resulting in the formation of small fluid-filled vacuoles 250 at the interface 225. If the suction force is maintained, the small vacuoles 250 continue to grow and merge - as shown in Figure 2C - such that a blister 255 is formed at the interface 225 between the epidermis layer 220 and the dermis layer 230. Such damaging as shown in Figures 2B and 2C activates the natural re-epithelialization process to generate new epidermal tissue which results in skin rejuvenation.
The skin treatment may be stopped at the application time interval Δt of Figure 2B, in which case blister 255 formation is prevented but still damage is induced at the interface 225 to initiate the rejuvenation process. A benefit of stopping the skin rejuvenation treatment before the blister 255 of Figure 2C is formed resides in that the damaging of the interface 225 between the epidermis layer 220 and the dermis layer 230 is hardly visible - causing little cosmetic discomfort. Still, the damage to the interface 225 as shown in Figure 2B is sufficient to initiate the rejuvenation process. Furthermore, the overall treatment time is reduced when the treatment is stopped in the situation shown in Figure 2B, because the damage shown in Figure 2B already occurs at between 30% and 70% of the time required to generate a reference blister formation 255, for example, the blister shown in Figure 2C. A reference blister-time Tb is a time interval during which, using a predetermined area of the opening 120 and using a predefined level of treatment pressure Pt in the suction chamber 110, the treatment pressure Pt should be applied for creating the reference blister formation 255. Thus, choosing the application time interval Δt to be within a range between 30% and 70% of a reference blister-time Tb will induce sufficient damage at the interface to initiate skin rejuvenation while avoiding cosmetic effects such as blisters. There are several parameters which may be sensed by the non-invasive device 100 according to the invention to measure whether the initial damage or separation of the interface 225 as shown in Figure 2B is occurring at the treatment location. For example, a pressure sensor 150 (see Figure 3) may be used in the non-invasive device 100 to sense whether the pressure inside the suction chamber 110 changes almost stepwise (shown in Figure 3B). Such a rapid change in pressure may result from initial ruptures and tears in the interface 225, causing vacuoles 250, and may be used as indicator, for example, to stop the treatment before a blister 255 is generated. Alternatively, an optical sensor 150 or acoustic sensor 150 may be used to sense a parameter associated with this initial damage or separation of the interface 225 to ensure that the treatment is stopped before a blister 255 is generated. Of course, alternatively, the skin treatment may continue until the application time interval Δt is equal to the reference blister-time Tb required to generate the blister 255: Δt = Tb. In any of the application time intervals Δt shown in Figures 2B or 2C, the outer skin 210 of the skin tissue 200 remains intact ,without the treatment causing physical rupture or tearing of the skin surface 210 - and hence any risk of infections and complications is reduced significantly.

Figure 3A shows a detail of the non-invasive device 100 according to the invention, in which the non-invasive device comprises a sensor 150 for sensing a parameter associated with the damaging or separation of the interface 225 between the epidermis layer 220 and the dermis layer 230. In the details shown in Figure 3A, the damage at the interface 225 has led to a full blister 255 while in an alternative embodiment of the non-invasive device 100 according to the invention the sensor 150 is used to stop the treatment before a full blister 225 is generated. Figure 3A again shows the opening 120, together with the stratum corneum 240, the epidermis layer 220 and the dermis layer 230. The sensor 150 is schematically applied to a side wall of the opening 120 but may be applied at different locations as long as it senses a parameter associated with the damaging of the interface 225.

The sensor 150 may, for example, be a pressure sensor 150 sensing a pressure inside the suction chamber 110 of the non-invasive device 100 according to the invention. Alternatively, the sensor 150 may, for example, be an optical sensor 150. Such an optical sensor 150 may, for example, identify a relatively fast change of the outer surface 210 of the skin tissue 200, which may be caused by the initial damage of the interface 225, allowing a user to stop the treatment before the blister 255 is created at the treatment location. The optical sensor 150 may alternatively be a microscope focusing at the interface 225 between the epidermis layer 220 and the dermis layer 230 to register the generation of vacuoles 250 due to the initial damage at the interface 225. Even further alternatively, the sensor 150 may be an acoustic sensor 150 such as a piezo-electric acoustic sensor 150 sensing an acoustic wave propagating through the skin tissue 200 generated by the initial damage at the interface 225.

In the embodiment in which the sensor 150 is a pressure sensor, the pressure may change relatively rapidly due to the onset of damage at the interface 225. This is schematically shown in Figure 3B in which a variation of the pressure Pt inside the suction chamber 110 during treatment shows a stepwise behavior (indicated with the arrow inside the dashed circle). As indicated before, such a variation of the pressure typically corresponds to the initial damage in the form of tears and ruptures at the interface 225 from which relatively small vacuoles 250 are created as shown in Figure 2B. When such a stepwise change in the pressure Pt is being sensed, the treatment may be stopped, which results in the absence of visible blisters 255 at the outer surface 210 of the skin tissue 200 while the rejuvenation process is still initiated.

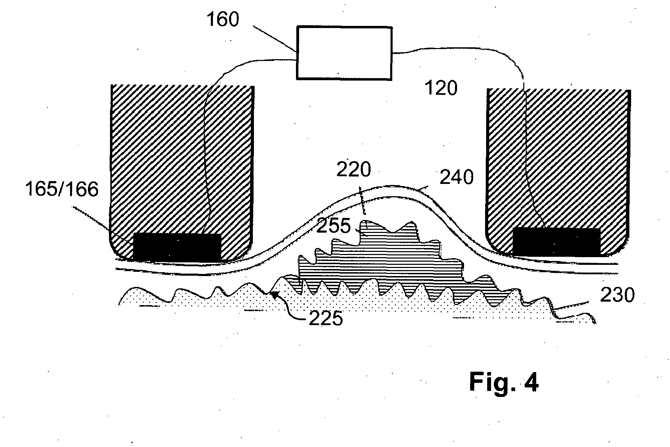

Figure 4 shows a detail of the non-invasive device 100 according to the invention in which the non-invasive device 100 comprises a heating element 165 or cooling element 166. The heating element 165 or cooling element 166 may be controlled by an additional heating controller 160 which preferably is connected to the controller 140. Alternatively, the controller 140 is directly connected to the heating element 165 or cooling element 166 for directly controlling the heating element 165 or cooling element 166. The increase of the temperature at or near the interface 225 also reduces the overall treatment time required to achieve damage at the interface 225 to initiate the rejuvenation process. An increase of the temperature to 40 or 42 degrees Celsius would significantly reduce the treatment time. A non-exhaustive listing of examples of different heating elements 165 that may be used are a Radio-Frequency (RF) heating element 165, a laser heating element 165, a Light Emitting Diode heating element 165, a contact heating element 165, and a Peltier heating element 165. Of course also other heating elements may be used. Furthermore, the heating element 165 may be configured to increase the temperature in the skin tissue 200 such that the epidermis layer 220, dermis layer 230, or the interface 225 between the epidermis layer 220 and the dermis layer 230 is thermally damaged. So, rather than reducing the treatment time of the non-invasive device 100 according to the invention, the use of such a heating element 165 directly causes thermal damage in the epidermis layer 220, dermis layer 230, or at the interface 225 to initiate the rejuvenation process. Temperature increases between 55°C up to 80°C should be achieved for that purpose during a specific time interval. Thermal damage is usually associated with a so-called damage integral, where the duration of the temperature applied is also important. In such an embodiment, the rejuvenation may, for example, be initiated both by using the suction force applied from the suction chamber 110 and the thermal damage induced by the heating element. Such thermal damage may be located deeper inside the skin tissue 200 and/or further into surrounding areas, and may complement the suction treatment.

Alternatively, the non-invasive device 100 comprises the cooling element 166 for reducing a temperature in the skin tissue 200 at or near the interface 225. Such a cooling element 166 may, for example, be a Peltier cooling element 166 for cooling the interface to a temperature ranging between -20 and -30 degrees Celsius. Also a reduction of the temperature at or near the interface 225 would reduce the time required to induce sufficient damage at the interface 225 to initiate skin rejuvenation. This reduction of the time necessary for the rejuvenation treatment, using the heating element 165 or the cooling element 166, may be experimentally determined and/or may be included in a look-up-table.

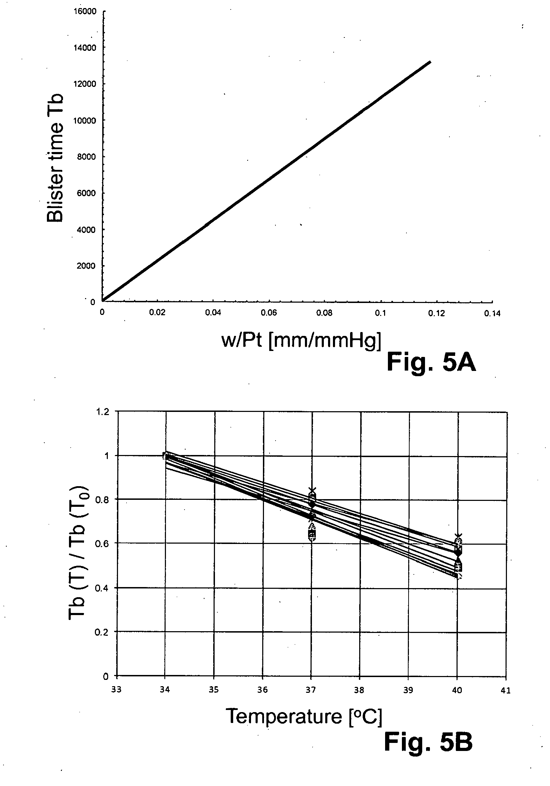

Figure 5A shows a graph indicating the blister time Tb required for different opening widths w and treatment pressure levels Pt, and Figure 5B shows a graph indicating the blister time Tb for different temperatures. From Figure 5A it may be seen that a reduction of the width w of the opening 120 and/or an increase of the level of the treatment pressure Pt reduce the blister time Tb and hence reduce the time required for the non-invasive device 100 to initiate skin rejuvenation. From Figure 5B it may be seen that an increase of the temperature also causes a reduction of the blister time Tb. In Figure 5B the blister time Tb at a certain temperature T (indicated with Tb(T)) is compared with the blister time Tb at a reference temperature To (indicated with Tb(T₀)), wherein the reference temperatureT₀ is chosen to be 34 degrees Celsius. A similar graph as shown in Figure 5B may be generated for cooling the interface 225 to temperatures between -20 and -30 degrees Celsius.

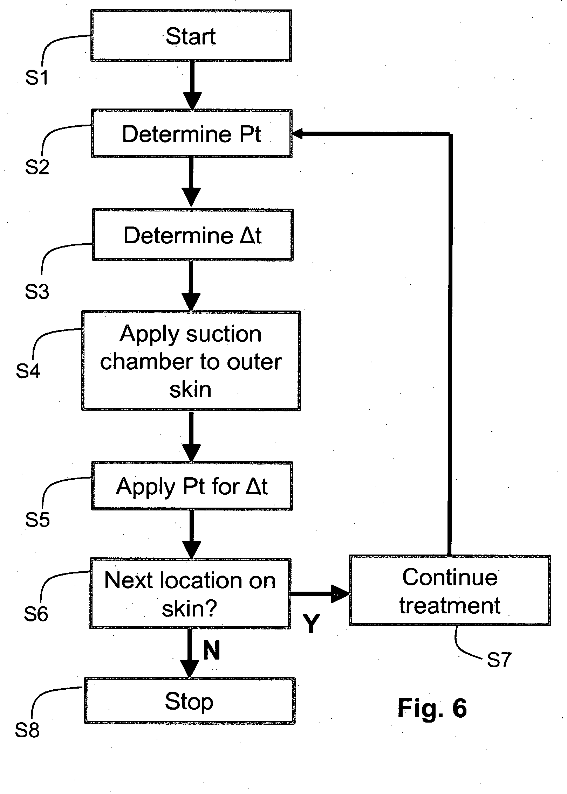

Figure 6 shows a flow diagram indicating the method according to the invention and a computer program product to perform the method according to the invention. The process starts at step S1, after which in step S2 a level of the treatment pressure Pt is determined for applying the rejuvenation process according to the invention. This level of treatment pressure Pt may, for example, be experimentally determined at the skin tissue of the person undergoing the treatment - for example, by determining the reference blister-time Tb at a certain part of the body and using a look-up-table to determine the preferred level of treatment pressure Pt to be applied for the treatment. In a next step S3, the application time interval Δt is determined. Also this application time interval Δt may be determined via a similar look-up-table or may be determined during the treatment using the sensor 150 as described earlier. Subsequently, the suction chamber 110 is applied to the outer skin 210 of the skin tissue 200 in step S4 and the determined level of treatment pressure Pt is applied via the openings 120 in the skin contact surface 115 of the non-invasive device 100 during the application time interval Δt in step S5. If the application time interval Δt has lapsed, the non-invasive device 100 according to the invention may generate a visible or audible alarm for the user to stop the treatment or, alternatively, the controller 140 may switch off the vacuum pump 130 to stop the treatment. Subsequently, if there is a next location in the skin tissue 200, step S6 will continue the treatment via step S7 to step S2 after which again the level of treatment pressure Pt is determined and subsequently the predetermined time delay Δt, etcetera. If there are no areas of the skin surface 210 that need further treatment, the process stops at step S8.

It will be appreciated that the invention also extends to computer programs, particularly computer programs on or in a carrier, adapted for putting the invention into practice. The program may be in the form of a source code, an object code, a code intermediate source and object code such as a partially compiled form, or in any other form suitable for use in the implementation of the method according to the invention. It will also be appreciated that such a program may have many different architectural designs. For example, a program code implementing the functionality of the method or system according to the invention may be subdivided into one or more subroutines. Many different ways to distribute the functionality among these subroutines will be apparent to the skilled person. The subroutines may be stored together in one executable file to form a self-contained program. Such an executable file may comprise computer executable instructions, for example processor instructions and/or interpreter instructions (e.g. Java interpreter instructions). Alternatively, one or more or all of the subroutines may be stored in at least one external library file and linked with a main program either statically or dynamically, e.g. at run-time. The main program contains at least one call to at least one of the subroutines. Also, the subroutines may comprise function calls to each other. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the means of at least one of the systems and/or products set forth. These instructions may be subdivided into subroutines and/or be stored in one or more files that may be linked statically or dynamically.

The carrier of a computer program may be any entity or device capable of carrying the program. For example, the carrier may include a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the program is embodied in such a signal, the carrier may be constituted by such a cable or other device or means. Alternatively, the carrier may be an integrated circuit in which the program is embedded, the integrated circuit being adapted for performing, or for use in the performance of, the relevant method.
It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably. programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A non-invasive device (100) for rejuvenation of skin tissue (200), the non-invasive device (100) comprising:
a suction chamber (110) having a skin contact surface (115) comprising an opening (120) for exposing the skin tissue (200) to a treatment pressure (Pt) present in the suction chamber (110) for applying a suction force to a portion of an outer surface (210) of the skin tissue (200) corresponding to an area of the opening (120), the treatment pressure (Pt) being lower than an ambient pressure outside the suction chamber, and the suction chamber (110) being dimensioned so as to allow it to be manually applied to the outer surface (210) of the skin tissue (200), and
a controller (140) for controlling a level of the treatment pressure (Pt) in the suction chamber (110) and for controlling an application time interval (Δt) of the treatment pressure (Pt),
**characterized in that** the controller (140) is configured for controlling the level of the treatment pressure (Pt) in the suction chamber (110) and for controlling the application time interval (Δt) of the treatment pressure (Pt) for damaging an interface (225) between an epidermis layer (220) and a dermis layer (230) of the skin tissue (200) to initiate rejuvenation of the skin tissue (200),
wherein the area of the opening (120) in the suction chamber (110) is less than 10 square millimeters.

2. The non-invasive device (100) as claimed in claim 1, wherein the opening (120) in the skin contact surface (115) of the suction chamber (110) is in a range between 4 square millimeters and 0.75 square millimeter.

3. The non-invasive device (100) as claimed in claim 1, wherein the level of the treatment pressure (Pt) in the suction chamber (110) is in a range between 0.1333x10⁵ and 1.2x10⁵ N/m² (between 10⁵ and 9x10⁵ milliTor) below ambient pressure.

4. The non-invasive device (100) as claimed in claim 1, wherein the application time interval (Δt) is in a range between 1 second and 10 minutes.

5. The non-invasive device (100) as claimed in claim 1, wherein the skin contact surface (115) comprises an array of openings (120), a distance (d) between two adjacent openings (120) in the array of openings (120) being larger than a width (w) of the opening (120).

6. The non-invasive device (100) as claimed in claim 1, wherein the controller (140) comprises a look-up-table for determining the level of the treatment pressure (Pt) and the application time interval (Δt) for a specific skin type.

7. The non-invasive device (100) as claimed in claim 1, wherein the non-invasive device (100) comprises a sensor (150) connected to the controller (140), the sensor (150) being configured for sensing a parameter associated with the damaging of the interface (225) between an epidermis layer (220) and a dermis layer (230) of the skin tissue (200).

8. The non-invasive device (100) as claimed in claim 7, wherein the sensor (150) is selected from the list comprising:
a pressure sensor (150) for sensing a pressure increase caused by the damaging of the interface (225),
an optical sensor (150) for sensing a change of the outer surface (210) of the skin tissue (200) caused by the damaging of the interface (225),
an optical sensor (150) for sensing a change in the interface (225) between the epidermis layer (220) and the dermis layer (230) caused by the damaging of said interface, and
an acoustic sensor (150) for sensing an acoustic signal caused by the damaging of, and emanating from, the interface (225) between the epidermis layer (220) and the dermis layer (230).

9. The non-invasive device (100) as claimed in any one of the preceding claims, wherein the controller (140) is configured to stop the application of the treatment pressure to the skin tissue (200) before a reference blisterformation (255) is formed at the interface (225) between the epidermis layer (220) and the dermis layer (230) in the skin tissue (200), the reference blister formation (255) corresponding to a blister formation comprising one or more blister cavities at the interface (225) between the epidermis layer (220) and the dermis layer (230) covering 80% of the area of the opening (120) in the suction chamber (110).

10. The non-invasive device (100) as claimed in claim 9, wherein the application time interval (Δt) ranges between 30% and 70% of a reference blister-time (Tb), wherein the reference blister-time (Tb) is a time interval during which, using a predetermined area of the opening (120) and using a predefined level of treatment pressure (Pt) in the suction chamber (110), the treatment pressure (Pt) should be applied for creating the reference blister formation(255).

11. The non-invasive device (100) as claimed in claim 1, wherein the non-invasive device (100) comprises a heating element (165) being configured for increasing a temperature of the skin tissue (200) at or near the interface (225) between the epidermis layer (220) and the dermis layer (230).

12. The non-invasive device (100) as claimed in claim 11, wherein the heating element (165) is selected from the list comprising:
a Radio-Frequency (RF) heating element (165),
a laser heating element (165),
a Light Emitting Diode heating element (165),
a contact heating element (165),
a Peltier heating element (165), and
a broadband light source.

13. The non-invasive device as claimed in claim 11, wherein the heating element (165) is configured for increasing the temperature of the skin tissue (200) to a level necessary for thermally damaging the epidermis layer (220), dermis layer (230) or the interface (225) between the epidermis layer (220) and the dermis layer (230).

14. The non-invasive device (100) as claimed in claim 1, wherein the non-invasive device (100) comprises a cooling element (166) being configured for reducing a temperature of the skin tissue (200) at or near the interface (225) between the epidermis layer (220) and the dermis layer (230).

15. A skin treatment method for rejuvenating skin tissue (200), the method comprising the steps of:
bringing a suction chamber (110) into contact with an outer surface of skin tissue (200), the suction chamber (110) having a skin contact surface (115) comprising an opening (120) for exposing the skin tissue (200) to a treatment pressure (Pt) present in the suction chamber (110) for applying a suction force to a portion of the outer surface (210) of the skin tissue (200) corresponding to an area of the opening (120), the treatment pressure (Pt) being lower than an ambient pressure outside the suction chamber, and the suction chamber (110) being dimensioned so as to allow it to be manually applied to the outer surface (210) of the skin tissue (200), and
applying a level of treatment pressure (Pt) during an application time interval (Δt) to the outer surface (210) of the skin tissue (200), using the suction chamber (110), **characterized in that** said applying the level of treatment pressure (Pt) during the application time interval (Δt) to the outer surface (210) of the skin tissue (200) is for damaging an interface (225) between an epidermis layer (220) and a dermis layer (230) of the skin tissue (200) to initiate rejuvenation of the skin tissue, wherein the area of the opening (120) in the suction chamber (110) is less than 10 square millimeters.

## Patentansprüche

1. Nichtinvasive Vorrichtung (100) zur Verjüngung des Hautgewebes (200), wobei die nichtinvasive Vorrichtung (100) Folgendes umfasst:
eine Saugkammer (110) mit einer Hautkontaktoberfläche (115), die eine Öffnung (120) umfasst, um das Hautgewebe (200) einem in der Saugkammer (110) vorhandenem Behandlungsdruck (Pt) zum Ausüben einer Saugkraft auf einen Teil einer Außenfläche (210) des Hautgewebes (200) auszusetzen, der einer Fläche der Öffnung (120) entspricht, wobei der Behandlungsdruck (Pt) niedriger als ein Umgebungsdruck außerhalb der Saugkammer ist, und wobei die Saugkammer (110) so dimensioniert ist, dass sie manuell auf die Außenfläche (210) des Hautgewebes (200) aufgelegt werden kann, und
eine Steuereinheit (140) zum Steuern eines Niveaus des Behandlungsdrucks (Pt) in der Saugkammer (110) und zum Steuern eines Ausübungszeitintervalls (Δt) des Behandlungsdrucks (Pt),
**dadurch gekennzeichnet, dass** die Steuereinheit (140) konfiguriert ist, um den Niveau des Behandlungsdrucks (Pt) in der Saugkammer (110) so zu steuern und das Ausübungszeitintervall (Δt) des Behandlungsdrucks (Pt) so zu steuern, dass eine Grenzfläche (225) zwischen einer Epidermisschicht (220) und einer Dermisschicht (230) des Hautgewebes (200) beschädigt wird, um eine Verjüngung des Hautgewebes (200) einzuleiten,
wobei die Fläche der Öffnung (120) in der Saugkammer (110) kleiner ist als 10 mm².

2. Nichtinvasive Vorrichtung (100) nach Anspruch 1, wobei die Öffnung (120) in der Hautkontaktoberfläche (115) der Saugkammer (110) in einem Bereich zwischen 4 mm² und 0,75 mm² liegt.

3. Nichtinvasive Vorrichtung (100) nach Anspruch 1, wobei das Niveau des Behandlungsdrucks (Pt) in der Saugkammer (110) in einem Bereich zwischen 0,1333 x 10⁵ und 1,2 x 10⁵ N/m² (zwischen 10⁵ und 9 x 10⁵ milliTor) unter dem Umgebungsdruck liegt.

4. Nichtinvasive Vorrichtung (100) nach Anspruch 1, wobei das Ausübungszeitintervall (Δt) in einem Bereich zwischen 1 Sekunde und 10 Minuten liegt.

5. Nichtinvasive Vorrichtung (100) nach Anspruch 1, wobei die Hautkontaktoberfläche (115) ein Array von Öffnungen (120) umfasst, wobei ein Abstand (d) zwischen zwei benachbarten Öffnungen (120) in dem Array von Öffnungen (120) größer ist als eine Breite (w) der Öffnung (120).

6. Nichtinvasive Vorrichtung (100) nach Anspruch 1, wobei die Steuereinheit (140) eine Nachschlagetabelle zum Ermitteln des Niveaus des Behandlungsdrucks (Pt) und des Ausübungszeitintervalls (Δt) für einen bestimmten Hauttyp umfasst.

7. Nichtinvasive Vorrichtung (100) nach Anspruch 1, wobei die nichtinvasive Vorrichtung (100) einen Sensor (150) umfasst, der mit der Steuereinheit (140) verbunden ist, wobei der Sensor (150) konfiguriert ist, um einen mit der Beschädigung der Grenzfläche (225) zwischen einer Epidermisschicht (220) und einer Dermisschicht (230) des Hautgewebes (200) zusammenhängenden Parameter zu erfassen.

8. Nichtinvasive Vorrichtung (100) nach Anspruch 7, wobei der Sensor (150) ausgewählt wird aus der Liste umfassend:
einen Drucksensor (150) zum Erfassen des durch die Beschädigung der Grenzfläche (225) verursachten Druckanstiegs,
einen optischen Sensor (150) zum Erfassen einer durch die Beschädigung der Grenzfläche (225) verursachten Veränderung der Außenfläche (210) des Hautgewebes (200);
einen optischen Sensor (150) zum Erfassen einer durch die Beschädigung der genannten Grenzfläche verursachten Veränderung in der Grenzfläche (225) zwischen der Epidermisschicht (220) und der Dermisschicht (230), und
einen akustischen Sensor (150) zum Erfassen eines Signals, das durch die Beschädigung der Grenzfläche (225) zwischen der Epidermisschicht (220) und der Dermisschicht (230) verursacht wird und von der Grenzfläche ausgestrahlt wird.

9. Nichtinvasive Vorrichtung (100) nach einem der vorhergehenden Ansprüche, wobei die Steuereinheit (140) konfiguriert ist, um die Ausübung des Behandlungsdrucks auf das Hautgewebe (200) zu stoppen, bevor eine Referenzblasenbildung (255) an der Grenzfläche (225) zwischen der Epidermisschicht (220) und der Dermisschicht (230) in dem Hautgewebe (200) entsteht, wobei die Referenzblasenbildung (255) einer Blasenbildung entspricht, die eine oder mehrere Blasenkavitäten an der Grenzfläche (225) zwischen der Epidermisschicht (220) und der Dermisschicht (230) umfasst, welche 80 % der Fläche der Öffnung (120) in der Saugkammer (110) abdecken.

10. Nichtinvasive Vorrichtung (100) nach Anspruch 9, wobei das Ausübungszeitintervall (Δt) zwischen 30 % und 70 % einer Referenzblasenzeit (Tb) liegt, wobei die Referenzblasenzeit (Tb) ein Zeitintervall ist, in dem der Behandlungsdruck (Pt) unter Verwendung einer vorgegebenen Fläche der Öffnung (120) und unter Verwendung eines vorgegebenen Niveaus des Behandlungsdrucks (Pt) in der Saugkammer (110) ausgeübt werden sollte, um die Referenzblasenbildung (255) zu schaffen.

11. Nichtinvasive Vorrichtung (100) nach Anspruch 1, wobei die nichtinvasive Vorrichtung (100) ein Heizelement (165) umfasst, das konfiguriert ist, um eine Temperatur des Hautgewebes (200) an oder nahe der Grenzfläche (225) zwischen der Epidermisschicht (220) und der Dermisschicht (230) zu erhöhen.

12. Nichtinvasive Vorrichtung (100) nach Anspruch 11, wobei das Heizelement (165) ausgewählt wird aus der Liste umfassend:
ein Hochfrequenz- (HF) Heizelement (165),
ein Laser-Heizelement (165),
ein Leuchtdioden-Heizelement (165),
ein Kontakt-Heizelement (165),
ein Peltier-Heizelement (165) und
eine Breitband-Lichtquelle.

13. Nichtinvasive Vorrichtung nach Anspruch 11, wobei das Heizelement (165) konfiguriert ist, um die Temperatur des Hautgewebes (200) auf ein Niveau zu erhöhen, das für eine thermische Beschädigung der Epidermisschicht (220), der Dermisschicht (230), oder der Grenzfläche (225) zwischen der Epidermisschicht (220) und der Dermisschicht (230) erforderlich ist.

14. Nichtinvasive Vorrichtung (100) nach Anspruch 1, wobei die nichtinvasive Vorrichtung (100) ein Kühlelement (166) umfasst, das konfiguriert ist, um eine Temperatur des Hautgewebes (200) an oder nahe der Grenzfläche (225) zwischen der Epidermisschicht (220) und der Dermisschicht (230) zu verringern.

15. Hautbehandlungsverfahren zur Verjüngung des Hautgewebes (200), wobei das Verfahren die folgenden Schritte umfasst:
Inkontaktbringen einer Saugkammer (110) mit einer Außenfläche des Hautgewebes (200), wobei die Saugkammer (110) eine Hautkontaktoberfläche (115) hat, die eine Öffnung (120) umfasst, um das Hautgewebe (200) einem in der Saugkammer (110) vorhandenem Behandlungsdruck (Pt) zum Ausüben einer Saugkraft auf einen Teil einer Außenfläche (210) des Hautgewebes (200) auszusetzen, der einer Fläche der Öffnung (120) entspricht, wobei der Behandlungsdruck (Pt) niedriger als ein Umgebungsdruck außerhalb der Saugkammer ist, und wobei die Saugkammer (110) so dimensioniert ist, dass sie manuell auf die Außenfläche (210) des Hautgewebes (200) aufgelegt werden kann, und
Ausüben eines Behandlungsdruckniveaus (Pt) während eines Ausübungszeitintervalls (Δt) auf die Außenfläche (210) des Hautgewebes (200) unter Verwendung der Saugkammer (110), **dadurch gekennzeichnet, dass** das genannte Ausüben des Behandlungsdruckniveaus (Pt) während des Ausübungszeitintervalls (Δt) auf die Außenfläche (210) des Hautgewebes (200) dazu dient, eine Grenzfläche (225) zwischen einer Epidermisschicht (220) und einer Dermisschicht (230) des Hautgewebes (200) zu beschädigen, um eine Verjüngung des Hautgewebes einzuleiten, wobei die Fläche der Öffnung (120) in der Saugkammer (110) kleiner ist als 10 mm².

## Revendications

1. Dispositif non effractif (100) pour rajeunissement de tissu de peau (200), le dispositif non effractif (100) comprenant :
une chambre d'aspiration (110) possédant une surface de contact de peau (115) comprenant une ouverture (120) pour exposer le tissu de peau (200) à une pression de traitement (Pt) présente dans la chambre d'aspiration (110) pour appliquer une force d'aspiration sur une portion d'une surface extérieure (210) du tissu de peau (200) correspondant à une superficie de l'ouverture (120), la pression de traitement (Pt) étant inférieure à une pression ambiante à l'extérieur de la chambre d'aspiration, et la chambre d'aspiration (110) étant dimensionnée afin de lui permettre d'être appliquée manuellement sur la surface extérieure (210) du tissu de peau (200), et
un dispositif de commande (140) pour commander un niveau de la pression de traitement (Pt) dans la chambre d'aspiration (110) et pour commander un intervalle de temps d'application (Δt) de la pression de traitement (Pt),
**caractérisé en ce que** le dispositif de commande (140) est configuré pour commander le niveau de la pression de traitement (Pt) dans la chambre d'aspiration (110) et pour commander l'intervalle de temps d'application (Δt) de la pression de traitement (Pt) pour endommager une interface (225) entre une couche d'épiderme (220) et une couche de derme (230) du tissu de peau (200) pour commencer le rajeunissement du tissu de peau (200),
dans lequel la superficie de l'ouverture (120) dans la chambre d'aspiration (110) est inférieure à 10 millimètres carrés.

2. Dispositif non effractif (100) selon la revendication 1, dans lequel l'ouverture (120) dans la surface de contact de peau (115) de la chambre d'aspiration (110) est dans une plage entre 4 millimètres carrés et 0,75 millimètre carré.

3. Dispositif non effractif (100) selon la revendication 1, dans lequel le niveau de la pression de traitement (Pt) dans la chambre d'aspiration (110) est dans une plage entre 0,1333 x 10⁵ et 1,2 x 10⁵N/m² (entre 10⁵ et 9 x 10⁵ millitor) en dessous de la pression ambiante.

4. Dispositif non effractif (100) selon la revendication 1, dans lequel l'intervalle de temps d'application (Δt) est dans une plage entre 1 seconde et 10 minutes.

5. Dispositif non effractif (100) selon la revendication 1, dans lequel la surface de contact de peau (115) comprend un réseau d'ouvertures (120), une distance (d) entre deux ouvertures adjacentes (120) dans le réseau d'ouvertures (120) étant supérieure à une largeur (w) de l'ouverture (120).

6. Dispositif non effractif (100) selon la revendication 1, dans lequel le dispositif de commande (140) comprend une table de conversion pour déterminer le niveau de la pression de traitement (Pt) et l'intervalle de temps d'application (Δt) pour un type de peau spécifique.

7. Dispositif non effractif (100) selon la revendication 1, dans lequel le dispositif non effractif (100) comprend un capteur (150) connecté au dispositif de commande (140), le capteur (150) étant configuré pour détecter un paramètre associé à l'endommagement de l'interface (225) entre une couche d'épiderme (220) et une couche de derme (230) du tissu de peau (200).

8. Dispositif non effractif (100) selon la revendication 7, dans lequel le capteur (150) est sélectionné parmi la liste comprenant :
un capteur de pression (150) pour détecter une augmentation de pression causée par l'endommagement de l'interface (225),
un capteur optique (150) pour détecter un changement de la surface extérieure (210) du tissu de peau (200) causé par l'endommagement de l'interface (225),
un capteur optique (150) pour détecter un changement de l'interface (225) entre la couche d'épiderme (220) et la couche de derme (230) causé par l'endommagement de ladite interface, et
un capteur acoustique (150) pour détecter un signal acoustique causé par l'endommagement de, et provenant de, l'interface (225) entre la couche d'épiderme (220) et la couche de derme (230).

9. Dispositif non effractif (100) selon l'une quelconque des revendications précédentes, dans lequel le dispositif de commande (140) est configuré pour arrêter l'application de la pression de traitement sur le tissu de peau (200) avant qu'une formation de cloque de référence (255) soit formée à l'interface (225) entre la couche d'épiderme (220) et la couche de derme (230) dans le tissu de peau (200), la formation de cloque de référence (255) correspondant à une formation de cloque comprenant une ou plusieurs cavités de cloque à l'interface (225) entre la couche d'épiderme (220) et la couche de derme (230) couvrant 80 % de la superficie de l'ouverture (120) dans la chambre d'aspiration (110).

10. Dispositif non effractif (100) selon la revendication 9, dans lequel l'intervalle de temps d'application (Δt) varie entre 30 % et 70 % d'un temps de cloque de référence (Tb), dans lequel le temps de cloque de référence (Tb) est un intervalle de temps durant lequel, en utilisant une superficie prédéterminée de l'ouverture (120) et en utilisant un niveau prédéfini de pression de traitement (Pt) dans la chambre d'aspiration (110), la pression de traitement (Pt) doit être appliquée pour créer la formation de cloque de référence (255).

11. Dispositif non effractif (100) selon la revendication 1, dans lequel le dispositif non effractif (100) comprend un élément chauffant (165) configuré pour augmenter une température du tissu de peau (200) à ou près de l'interface (225) entre la couche d'épiderme (220) et la couche de derme (230).

12. Dispositif non effractif (100) selon la revendication 11, dans lequel l'élément chauffant (165) est sélectionné parmi la liste comprenant :
un élément chauffant à radiofréquence (RF) (165),
un élément chauffant à laser (165),
un élément chauffant à diode électroluminescente (165),
un élément chauffant à contact (165),
un élément chauffant à effet Peltier (165), et
une source de lumière à large bande.

13. Dispositif non effractif selon la revendication 11, dans lequel l'élément chauffant (165) est configuré pour augmenter la température du tissu de peau (200) jusqu'à un niveau nécessaire pour thermiquement endommager la couche d'épiderme (220), la couche de derme (230) ou l'interface (225) entre la couche d'épiderme (220) et la couche de derme (230).

14. Dispositif non effractif (100) selon la revendication 1, dans lequel le dispositif non effractif (100) comprend un élément refroidissant (166) configuré pour réduire une température du tissu de peau (200) à ou près de l'interface (225) entre la couche d'épiderme (220) et la couche de derme (230).

15. Procédé de traitement de peau pour rajeunir un tissu de peau (200), le procédé comprenant les étapes de :
la mise d'une chambre d'aspiration (110) en contact avec une surface extérieure de tissu de peau (200), la chambre d'aspiration (110) possédant une surface de contact de peau (115) comprenant une ouverture (120) pour exposer le tissu de peau (200) à une pression de traitement (Pt) présente dans la chambre d'aspiration (110) pour appliquer une force d'aspiration sur une portion de la surface extérieure (210) du tissu de peau (200) correspondant à une superficie de l'ouverture (120), la pression de traitement (Pt) étant inférieure à une pression ambiante à l'extérieur de la chambre d'aspiration, et la chambre d'aspiration (110) étant dimensionnée afin de lui permettre d'être appliquée manuellement sur la surface extérieure (210) du tissu de peau (200), et
l'application d'un niveau de pression de traitement (Pt) durant un intervalle de temps d'application (Δt) sur la surface extérieure (210) du tissu de peau (200), en utilisant la chambre d'aspiration (110), **caractérisé en ce que** ladite application du niveau de pression de traitement (Pt) durant l'intervalle de temps d'application (Δt) sur la surface extérieure (210) du tissu de peau (200) est pour endommager une interface (225) entre une couche d'épiderme (220) et une couche de derme (230) du tissu de peau (200) pour commencer un rajeunissement du tissu de peau, dans lequel la superficie de l'ouverture (120) dans la chambre d'aspiration (110) est inférieure à 10 millimètres carrés.
